Europäisches Patentamt
European Patent Office
Office européen des brevets

(19)

(11) Veröffentlichungsnummer: **0 379 006 A2**

(12) **EUROPÄISCHE PATENTANMELDUNG**

(21) Anmeldenummer: **90100192.5**

(51) Int. Cl.⁵: **C07C 217/90, C07C 217/58**

(22) Anmeldetag: **05.01.90**

(30) Priorität: **18.01.89 DE 3901272**
**28.06.89 DE 3921165**

(43) Veröffentlichungstag der Anmeldung:
**25.07.90 Patentblatt 90/30**

(84) Benannte Vertragsstaaten:
**BE DE ES FR GB IT NL**

(71) Anmelder: **BAYER AG**

**D-5090 Leverkusen 1 Bayerwerk(DE)**

(72) Erfinder: **Müller, Hanns-Peter, Dr.**
**Weizenfeld 36**
**D-5060 Bergisch Gladbach 2(DE)**
Erfinder: **Reuter, Knud, Dr.**
**Scheiblerstrasse 99**
**D-4150 Krefeld(DE)**
Erfinder: **Weymanns, Dr.**
**Karl-Arnold-Strasse 4**
**D-5090 Leverkusen 1(DE)**
Erfinder: **Mayska, Paul Johannes, Dr.**
**Bodelschwinghstrasse 18**
**D-4150 Krefeld(DE)**
Erfinder: **Freitag, Dieter, Dr.**
**Hasenheide 10**
**D-4150 Krefeld(DE)**
Erfinder: **Idel, Karsten-Josef, Dr.**
**Am Schwarzkamp 38**
**D-4150 Krefeld(DE)**
Erfinder: **Eckhardt, Volker, Dr.**
**Bodelschwinghstrasse 14**
**D-4150 Krefeld(DE)**
Erfinder: **Westeppe, Uwe, Dr.**
**Vogelskamp 72**
**D-4020 Mettmann(DE)**

(54) **Aminoarylether.**

(57) Aminoarylether, ein Verfahren zu ihrer Herstellung und ihre Verwendung zur Herstellung von Kunststoffen und Kunststoffmischungen.

Die Aminoarylether entsprechen der Formel (I)

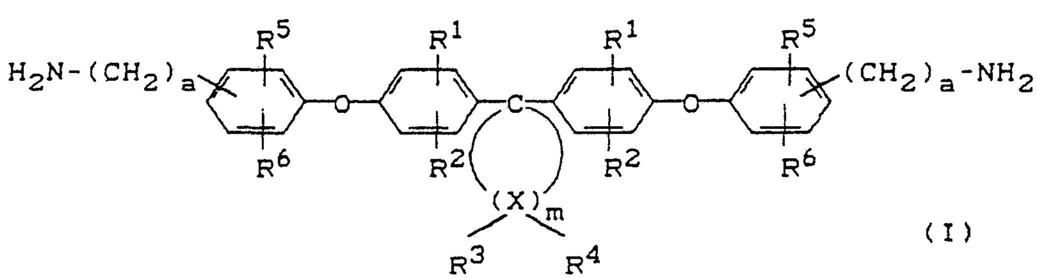

(I)

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl und $C_7$-$C_{12}$-Aralkyl,

$R^3$ und $R^4$, für jedes X individuell wählbar, unabhängig voneinander Wasserstoff und $C_1$-$C_{12}$-Alkyl und $C_5$-$C_{12}$-Cycloalkyl,

X Kohlenstoff

bedeuten,

mit der Maßgabe, daß an mindestens einem Atom X, $R^2$ und $R^4$ gleichzeitig Alkyl ist, worin ferner

EP 0 379 006 A2

$R^5$ und $R^6$ unabhängig voneinander Wasserstoff, $C_1$-$C_8$-Alkyl, $C_5$-$C_6$-Cycloalkyl, $C_6$-$C_{10}$-Aryl oder $C_7$-$C_{12}$-Aralkyl bedeuten und
m eine ganze Zahl von 4 bis 7 und
a 0 oder 1 ist.

## Aminoarylether

Die Erfindung betrifft Aminoarylether, ein Verfahren zu ihrer Herstellung sowie ihre Verwendung zur Herstellung von Kunststoffen und Kunststoffmischungen.

Gegenstand der Erfindung sind Aminoarylether der Formel (I)

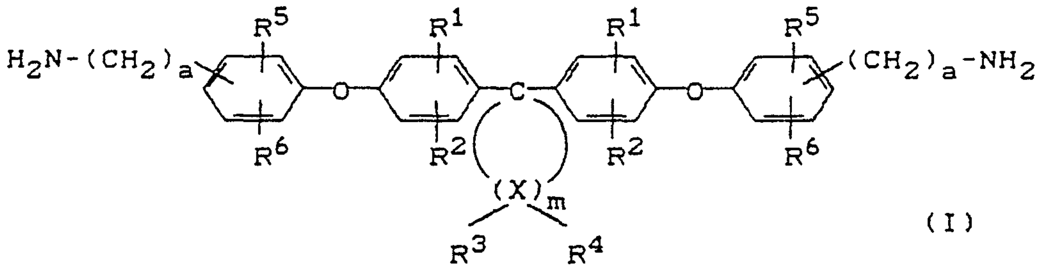

(I)

worin

$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl und $C_7$-$C_{12}$-Aralkyl,

$R^3$ und $R^4$, für jedes X individuell wählbar, unabhängig voneinander Wasserstoff, $C_1$-$C_{12}$-Alkyl und $C_5$-$C_{12}$-Cycloalkyl

X Kohlenstoff

bedeuten,

mit der Maßgabe, daß an mindestens einem Atom X, $R^2$ und $R^4$ gleichzeitig Alkyl ist, worin ferner

$R^5$ und $R^6$ unabhängig voneinander Wasserstoff, $C_1$-$C_8$-Alkyl, $C_5$-$C_6$-Cycloalkyl, $C_6$-$C_{10}$-Aryl oder $C_7$-$C_{12}$-Aralkyl bedeuten

m eine ganze Zahl von 4 bis 7 und

a 0 oder 1 ist.

Halogen in Formel (I) ist Fluor, Chlor und Brom, insbesondere Brom und Chlor; Alkyl ist bevorzugt Methyl, Ethyl, Propyl und Butyl, besonders bevorzugt Methyl; Cycloalkyl ist bevorzugt Cyclopentyl und Cyclohexyl, besonders bevorzugt Cyclohexyl; Aryl ist bevorzugt Phenyl und Naphthyl, besonders bevorzugt Phenyl und Aralkyl Benzyl.

In der Formel (I) steht m bevorzugt für 4 und 5, insbesondere für 5.

Bevorzugte erfindungsgemäße Aminoarylether sind solche der Formeln

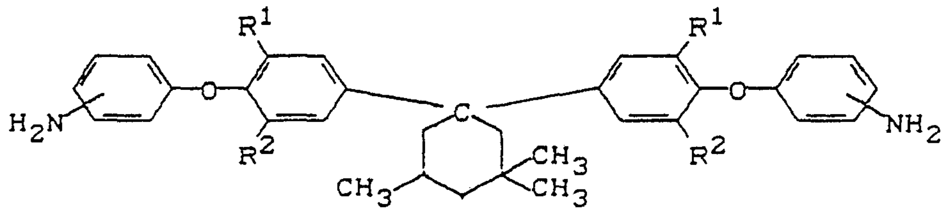

(II)

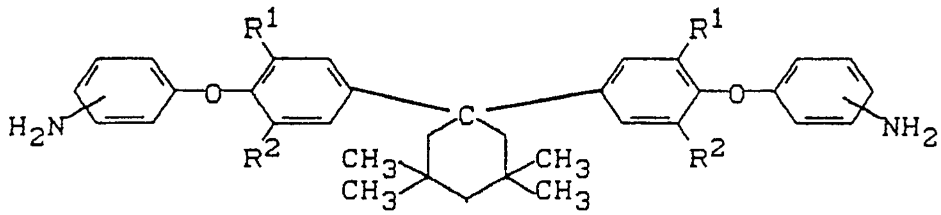

(III)

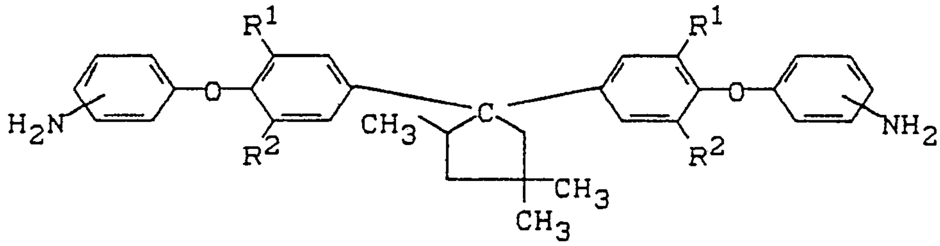

(IV)

worin $R^1$ und $R^2$ die für Formel (I) genannte Bedeutung besitzen.

Bevorzugt sind Aminoarylether der Formel (II), wenn $R^1$ und $R^2$ unabhängig voneinander Wasserstoff oder Methyl bedeuten, also insbesondere der Aminoarylether der Formel (IIa)

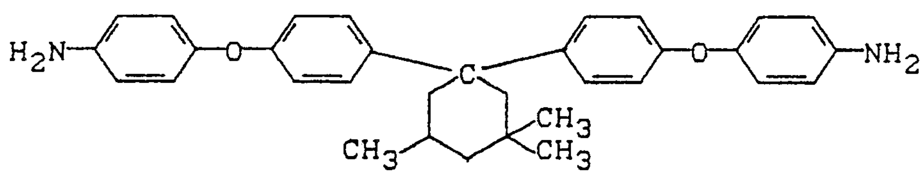

(IIa)

Ein weiterer Gegenstand der Erfindung ist ein Verfahren zur Herstellung von Aminoarylethern der Formel (I), das dadurch gekennzeichnet ist, daß man Diphenole der Formel (V),

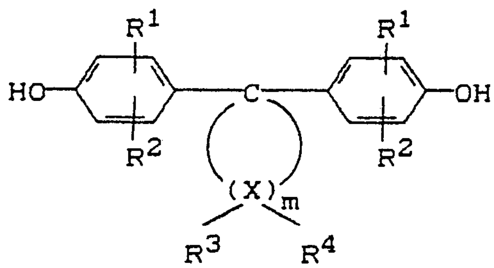

(V)

worin
$R^1$, $R^2$, $R^3$, $R^4$, X und m, wie sie in Formel (I) definiert sind, bevorzugt in Form ihrer Alkalisalze, mit Nitroverbindungen der Formel (VI)

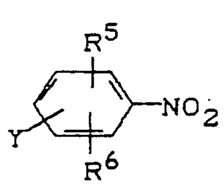

(VI) oder Nitril-Verbindungen der Formel (VII)

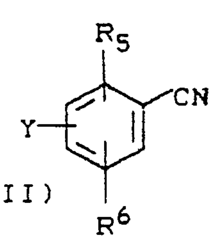

(VII)

worin
$R^5$ und $R^6$ wie in Formel (I) definiert sind und
Y Halogen, bevorzugt Chlor, oder die Nitrogruppe bedeutet,
umsetzt und die gebildeten Nitro- bzw. Cyanoarylether zu den Aminoverbindungen reduziert.

Bevorzugt werden die Verbindungen (V) in Form ihrer Natrium- oder Kaliumsalze (die nicht isoliert werden müssen) in polaren aprotischen Lösungsmitteln, wie N-Methylpyrrolidon, N-Methylcaprolactam, N,N-Dimethylformamid, N,N-Dimethylacetamid, Dimethylsulfoxid, Dimethylsulfon, Sulfolan oder Tetramethylharnstoff, mit 2 bis 3 Mol, bezogen auf 1 Mol (V) der Nitroverbindungen (VI) oder Cyanoverbindungen (VII) bei 30 bis 180 °C, bevorzugt 60 bis 150 °C umgesetzt. Dabei können anteilig z.B. 0,1 bis 200 Gew.-%, bezogen auf das Gewicht des polaren Lösungsmittels, andere weniger polare Lösungsmittel mitverwendet werden, z.B. aromatische Kohlenwasserstoffe wie Toluol, Xylol, Mesitylen, Chlorbenzol oder aliphatische Kohlenwasserstoffe wie Benzin, Hexan, Heptan, Cyclohexan.

Die aromatischen Dihydroxyverbindungen der Formel (V) können in an sich bekannter Weise hergestellt werden durch Kondensation der entsprechenden Phenole mit den entsprechenden Ketonen in Gegenwart von sauren Katalysatoren sowie gegebenenfalls weiteren Cokatalysatoren. Verwiesen wird in diesem Zusammenhang auf die deutsche Patentanmeldung P 38 32 396.6 und auf Schnell, Chemistry and Physics of Polycarbonates, Interscience Publishers, New York, 1964.

Als in das erfindungsgemäße Verfahren einzusetzende Diphenole der Formel (V) kommen beispielsweise in Frage:

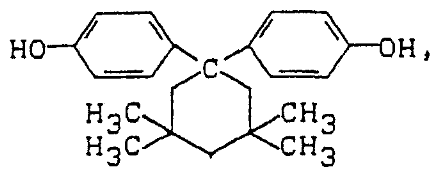

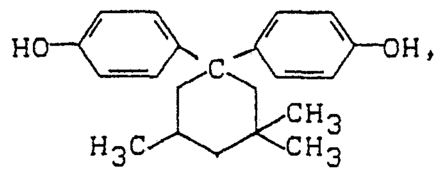

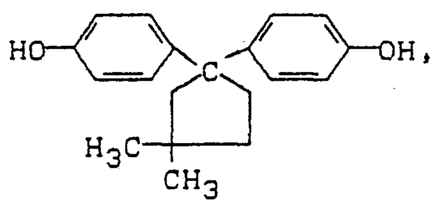

bevorzugt

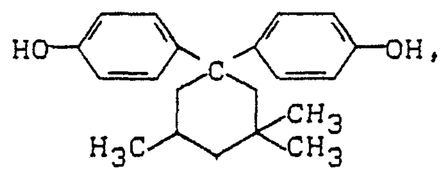

Als Verbindungen der Formel (VI) können z.B. eingesetzt werden:

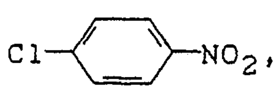

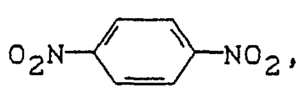

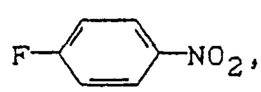

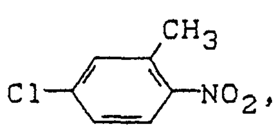

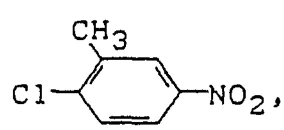

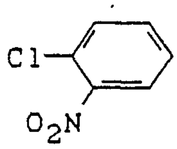

,

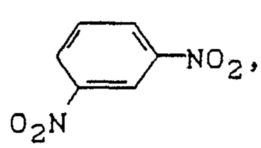

bevorzugt

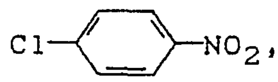,

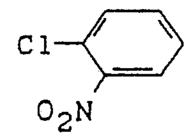,

besonders bevorzugt

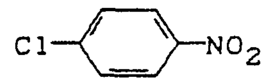.

Geeignete Nitrilverbindungen (VII) entsprechen diesen Formeln, nur ist $-NO_2$ durch -CN ersetzt.

Die Umsetzungsprodukte von (IV) und (VI) bzw. (VII) können z.B. in Lösungsmitteln wie Dimethylformamid, Dimethylacetamid, N-Methylpyrrolidon, Glykolmonomethylether oder Methanol bei 50 bis 80 °C und Drücken von 50 bis 100 bar mit Wasserstoff reduziert werden, wobei z.B. Raney-Nickel als Katalysator verwendet werden kann. Im Prinzip können bekannte Reduktionsverfahren für die Umwandlung von Nitroaromaten in Anilinderivate verwendet werden. (Siehe z.B. Organikum, 12. Aufl. 1973, S. 575-580; Houben-Weyl, Meth. d. Org. Chem. 4. Aufl. 1957, Bd. XI/1, Stickstoffverbind. II, Kap. IVa/2, Reduktion von Nitroverb., S. 360 bis 490).

Für Nitrilverbindungen wird bevorzugt Lithiumalumiumhydrid als Reduktionsmittel verwendet.

## Beispiel 1

93,1 g 1,1-(4,4′-Dihydroxydiphenyl)-3,3,5-trimethylcyclohexan, 53,2 g 45%ige NaOH, 400 ml Dimethylsulfoxid und 260 ml Toluol werden unter Stickstoff so lange am Wasserabscheider zum Rückfluß erhitzt, bis kein $H_2O$ mehr abgespalten wird. Anschließend wird eine Soxhlet-Apparatur aufgesetzt, die mit Molekularsieb 4 Å beschickt ist, und die Reaktionsmischung eine weitere Stunde zum Rückfluß erhitzt. Danach wird so lange Toluol abdestilliert, bis eine Innentemperatur von 145 °C erreicht ist. Zur abgekühlten Mischung werden 104 g 4-Chlornitrobenzol gegeben und die Reaktionsmischung 6 Stunden auf 100 °C erhitzt. Aus der wieder erkalteten Reaktionsmischung wird das auskristallisierte Reaktionsprodukt abgesaugt und mit Methanol und Wasser gewaschen. Man erhält 151,3 g der Verbindung

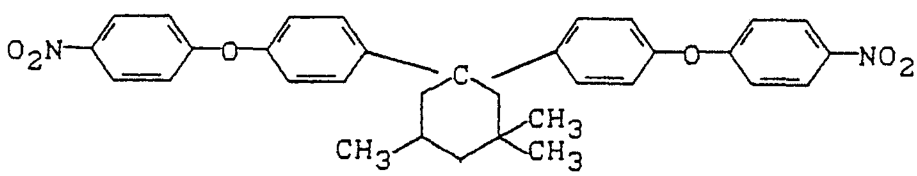

die durch ihr NMR-Spektrum und die Elementaranalyse (C 72,0 %, theor. 71,7 %; H 5,96 %, theor. 5,84 %; N 5,14 %, theor. 5,07 %) charakterisiert wird;
Schmp. 193 - 196 °C.

Reduktion der Dinitroverbindung zum Diaminoarylether: 144 g 1,1-(4,4′-Dinitrodiphenylether)-3,3,5-trimethylcyclohexan, 500 g Dimethylformamid und 25 g Raney-Nickel werden im 1,3 l-Autoklaven 5 Stunden auf 50 °C unter 50 bar Wasserstoff erhitzt.

Bei Zimmertemperatur wird der Katalysator abgetrennt und die Dimethylformamidlösung durch Einrühren in Eiswasser zur Kristallisation gebracht. Nach Aufarbeitung erhält man 124 g (97 % d. Th.) der Diaminoverbindung, die durch IR, NMR und GC-MS charakterisiert wurde, Schmp. 109 bis 112 °C.

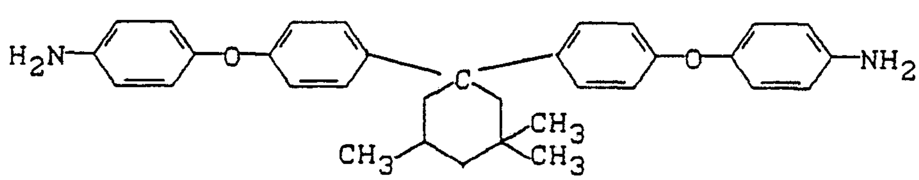

Beispiel 2

Wie in Beispiel 1 werden 23,2 g 1,1-(4,4′-Dihydroxydiphenyl)-3,3,5-trimethylcyclohexan, 13,4 g 45%ige NaOH, 100 ml Dimethylsulfoxid, 65 ml Toluol und 26 g 2-Chlornitrobenzol miteinander umgesetzt. Nach Abkühlen wird filtriert, die Mutterlauge mit Wasser versetzt und anschließend mit Methylenchlorid extrahiert. Die organische Phase wird eindestilliert; der Rückstand (23,4 g) besteht im wesentlichen aus

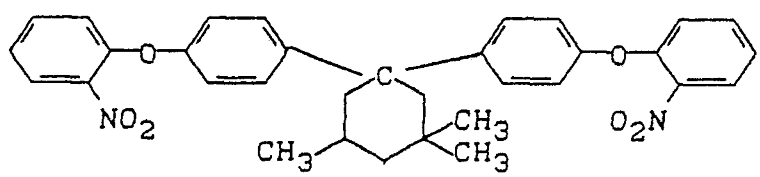

charakterisiert durch NMR-Spektrum und Elementaranalyse (C 71,7 %, theor. 71,7 %; H 5,95 %, theor. 5,84 %; N 5,12 %, theor. 5,07 %).

Die Dinitroverbindung wird wie in Beispiel 1 zum Diaminoarylether reduziert.

Beispiel 3

In einem 2 l Dreihalskolben, ausgerüstet mit Rührer, Thermometer, Überleitungsrohr, Rückflußkühler mit Calciumchlorid-Trockenrohr und Tropftrichter werden 600 ml trockenes Tetrahydrofuran (THF) und 4,18 g (0,11 mol) Lithiumaluminiumhydrid unter Stickstoff vorgelegt. Nach einigen Minuten wird eine Lösung aus 25,6 g (0,05 mol) 1,1-Bis-[4-(-cyanophenoxy)-phenyl]-3,3,5-trimethylcyclohexan in 100 ml trockenes THF in 30 Min. zugetropft. Nachdem die exotherme Reaktion beendet ist, werden noch 200 ml THF zugegeben und die Reaktionsmischung wird drei Stunden zum Sieden erhitzt. Anschließend wird auf 10° C abgekühlt und es werden 3,96 g (0,22 mol) dest. Wasser langsam zugetropft. (Achtung: Wasserstoffentwicklung) Der Feststoff wird abgesaugt und mit frischem THF extrahiert. Nach Abdestillieren des Lösungsmittels verbleibt ein Feststoff, der das gewünschte Produkt darstellt.
Ausbeute: 19,8 g (77 %).
Smp.: 25° C

**Ansprüche**

1. Aminoarylether der Formel (I)

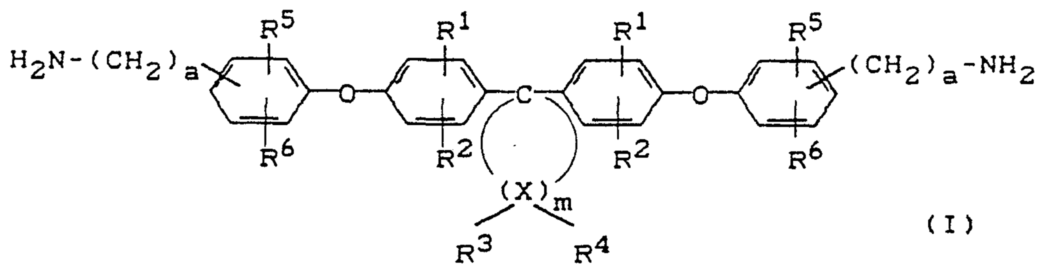

(I)

worin
$R^1$ und $R^2$ unabhängig voneinander Wasserstoff, Halogen, $C_1$-$C_8$-Alkyl, $C_5$-$C_6$-Cycloalkyl, $C_6$-$C_{10}$-Aryl und $C_7$-$C_{12}$-Aralkyl,
$R^3$ und $R^4$, für jedes X individuell wählbar, unabhängig voneinander Wasserstoff, $C_1$-$C_{12}$-Alkyl und $C_5$-$C_{12}$-Cycloalkyl,
X Kohlenstoff
bedeuten,
mit der Maßgabe, daß an mindestens einem Atom X, $R^2$ und $R^4$ gleichzeitig Alkyl ist, worin ferner
$R^5$ und $R^6$ unabhängig voneinander Wasserstoff, $C_1$-$C_8$-Alkyl, $C_5$-$C_6$-Cycloalkyl, $C_6$-$C_{10}$-Aryl oder $C_7$-$C_{12}$-Aralkyl bedeuten und

m eine ganze Zahl von 4 bis 7 und
a 0 oder 1 ist.

2. Verfahren zur Herstellung von Aminoaryl-cycloalkanen der Formel (I) des Anspruchs 1, dadurch gekennzeichnet, daß man Diphenole der Formel (V)

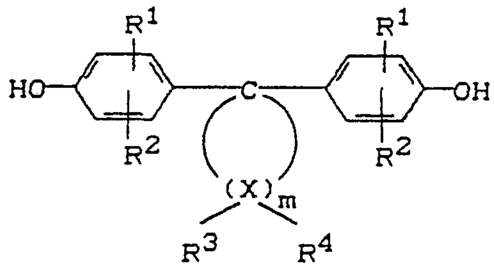

(V)

bevorzugt in Form ihrer Alkalisalze, worin
$R^1$, $R^2$, $R^3$, $R^4$, X und m die Bedeutung wie in Formel (I) in Anspruch 1 haben, mit Nitroverbindungen der Formel (VI)

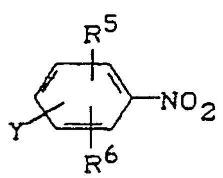

(VI) oder Nitril-Verbindungen

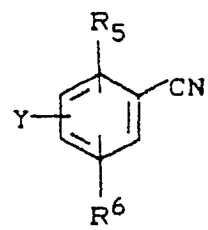

(VII)

worin
$R^5$ und $R^6$ die gleiche Bedeutung besitzen wie in Formel I und Y Halogen, bevorzugt Chlor, oder $NO_2$ bedeutet, umsetzt und die gebildete Nitro- bzw. Cyanoverbindung zum Diamin reduziert.

3. Verwendung der Aminoarylether nach Anspruch 1 zur Herstellung von Kunststoffen und Kunststoffmischungen.